# FASCICULE DE BREVET EUROPEEN

(11) **EP 2 509 458 B1**
(45) Date de publication et mention de la délivrance du brevet: **06.12.2017**
(21) Numéro de dépôt: 10790761.0
(22) Date de dépôt: 09.12.2010
(51) Int. Cl.: A61F 5/01

(54) **SEMELLE ORTHOPEDIQUE COMPRENANT DES MOYENS DE CORRECTION POUR LE TRAITEMENT DES METATARSALGIES ET DES AFFECTIONS CUTANEES PLANTAIRES**
ORTHOPÄDISCHE SOHLE MIT KORREKTURVORRICHTUNG ZUR BEHANDLUNG VON METATARSALGIE UND FUSSSOHLENHAUTERKRANKUNGEN
ORTHOPEDIC SOLE INCLUDING CORRECTIVE MEANS FOR TREATING METATARSALGIA AND PLANTAR SKIN DISORDERS

(30) Priorité: 09.12.2009 FR 0958812
(43) Date de publication de la demande: 17.10.2012
(73) Titulaire: Chenut, Pascal, 21220 Semezanges (FR)
(72) Inventeur: Chenut, Pascal, 21220 Semezanges (FR)
(74) Mandataire: Thomas, Nadine
(86) Numéro de dépôt international: PCT/EP2010/069315
(87) Numéro de publication internationale: WO 2011/070126

(56) Documents cités:
- EP-A1- 0 060 353
- FR-A1- 2 838 308
- FR-A1- 2 869 507
- FR-A7- 2 383 582
- US-A- 2 221 202
- US-A- 5 611 153
- US-A- 5 768 803

## Description

### DOMAINE DE L'INVENTION

La présente invention concerne une semelle orthopédique intérieure apte à être insérée dans une chaussure ou similaire pour soulager et/ou traiter des affections cutanées et/ou des durillons et/ou des douleurs métatarsiennes, destinée à toute personne ayant une affection cutanée et plus particulièrement aux sujets diabétiques afin d'éviter tout développement d'ulcération.

### ARRIERE-PLAN DE L'INVENTION

Dans le domaine de la podologie, il est bien connu que les durillons plantaires ou les affections cutanées peuvent être à l'origine de difficultés pour marcher et peuvent évoluer en ulcération en cas de déficience de la nutrition des tissus, tel que pour des patients atteints de diabète dit sucré de type 1 ou 2 notamment, ou pour des personnes âgées ayant des problèmes circulatoires.

Un durillon est classiquement une formation excessive de kératine normale sur le site corporel concerné. Son origine peut être soit liée à des troubles de type psoriasis, mycose ou eczéma par exemple, soit purement mécanique. La formation de durillons serait un processus normal permettant de protéger le pied des traumatismes. Les durillons plantaires se répartissent sur les têtes métatarsiennes et plus généralement sur la seconde tête métatarsienne.

La formation des durillons d'origine mécanique semble essentiellement due à des pressions excessives trop longues et trop importantes lors de la marche. En effet, l'augmentation de la pression stimule la libération de cytokines et de facteurs de croissance épidermique dans l'épiderme, entraînant la production accrue de cellules et finalement l'apparition de durillons. Ces excès de pression peuvent être dus à des anomalies anatomiques pouvant engendrer une modification de la démarche et un dysfonctionnement de la mécanique du pied, tels que des anomalies de l'arrière pied et de l'avant pied, des déformations digitales ou des excroissances osseuses par exemple, et/ou des facteurs extrinsèques telles que des chaussures mal ajustées, trop portées, inadaptées, etc., ou des activités sportives sporadiques ou intensives qui peuvent provoquer une charge excessive sur certaines parties du corps par exemple.

Le traitement des durillons consiste essentiellement dans l'application de crème à base d'acide acétylsalicylique (aspirine), d'acide lactique et d'urée sur le durillon. Parfois l'acide alpha-hydroxyle (provenant de la canne à sucre) est également préconisé. Par ailleurs, les durillons peuvent être traités par la pierre ponce qui doit être mouillée au préalable ou par tout autre dispositif abrasif.

On connaît, par ailleurs, des semelles dites intérieures, aptes à être insérées dans une chaussure, comportant des évidements et/ou des zones d'amortissement s'étendant à l'endroit du ou des durillons. C'est le cas notamment de la demande de brevet allemand DE 196 03 755, de la demande de brevet japonais JP 6054702, des demandes de brevet américain US 2001/0039746 et US 2005/0166425 et du brevet américain US 5,768,803 qui décrit notamment une semelle orthopédique selon le préambule de la revendication 1. Néanmoins, tous ces traitements et toutes ces semelles ne corrigent pas la cause de l'apparition des durillons de sorte que ces derniers réapparaissent régulièrement. De plus, malgré les traitements, il est parfois possible de voir survenir une suppuration liée à une infection de la bourse séreuse située en dessous du durillon pouvant entraîner une ulcération notamment chez les diabétiques.

On connaît également la demande de brevet français FR 2 869 507 qui décrit une orthèse plantaire à relief permettant prétendument de traiter la grande majorité des cas individuels. Ladite orthèse plantaire comporte, de l'avant vers l'arrière, sur une zone sensiblement plane, des zones dites sous-capitaux, à savoir une zone pour le premier métatarsien et une zone pour les quatrième et cinquième métatarsiens et, à l'arrière de ces zones, séparées l'une de l'autre, une zone de barre rétro-capitale ou appui rétro-capital et présentant un relief dont l'épaisseur varie de . La barre rétro-capitale est un élément situé juste en arrière des têtes métatarsiennes qui constituent pour l'ensemble la palette métatarsienne.

Ce type de semelle, outre le fait que l'épaisseur des éléments en relief comprise entre 1 et 10mm est trop grande procurant un inconfort trop important pour les patients ce qui rend ce type de semelle totalement inutilisable, ne permet pas de corriger la cause de l'apparition des durillons, la barre rétro-capitale étant positionnée en arrière des têtes métatarsiennes.

### BREVE DESCRIPTION DE L'INVENTION

L'un des buts de l'invention est donc de remédier à ces inconvénients en proposant une semelle orthopédique intérieure de conception simple et peu onéreuse, adaptée à la morphologie du pied de chaque patient et permettant de supprimer les excès de pression afin de supprimer les durillons et soulager les douleurs des patients.

A cet effet et conformément à l'invention, il est proposé une semelle orthopédique intérieure telle que définie dans la revendication 1, apte à être insérée dans une chaussure ou similaire pour soulager et/ou traiter des durillons et/ou des douleurs métatarsiennes ; ladite semelle est remarquable en ce qu'elle comporte au moins un élément de correction semi-rigide apte à procurer un appui dit rétro-capital interne, sur la partie arrière de la tête du premier métatarse, et/ou un appui rétro-capital dit médian, sur la partie arrière de la deuxième et/ou troisième et/ou quatrième tête métatarsienne, et/ou un appui rétro-capital dit externe, sur la partie arrière de la cinquième tête métatarsienne.

Conformément à l'invention, ledit élément de correction est positionné sur la face inférieure de ladite semelle.

Par ailleurs, ledit élément de correction est avantageusement solidarisé à la semelle de manière amovible.

Selon une variante d'exécution de la semelle, ledit élément de correction comporte des moyens de fixation aptes à coopérer avec des moyens de fixation complémentaires solidaires de la face inférieure de la semelle.

La semelle comprend sur sa face inférieure au moins un évidement s'étendant depuis le bord interne jusqu'au bord externe de la semelle, au droit des têtes métatarsiennes, et dans lequel l'élément de correction est susceptible d'être positionné.

Selon une autre variante d'exécution, ledit élément de correction comporte sur l'une de ses faces des bouclettes et/ou des crochets aptes à coopérer avec des crochets et/ou des bouclettes.

De manière avantageuse, la semelle comporte sur sa face inférieure un évidement positionné devant les têtes métatarsiennes, au niveau du capiton plantaire, ledit évidement s'étendant depuis le bord interne jusqu'au bord externe de la semelle, et apte à recevoir un élément dit antéro-capital inférieur comportant des lignes de prédécoupes afin de permettre la création d'au moins un creux sous le ou les durillons et/ou affections cutanées. Cela redonne de l'amorti situé à l'avant des têtes métatarsiennes et ceci, en association avec un capiton plantaire souple, optionnel, sous les têtes métatarsiennes à la face supérieure de la semelle. Ledit élément antéro-capital inférieur est prédécoupé en cinq zones pour dans certains cas créer un creux au niveau d'une ou plusieurs têtes métatarsiennes pour diminuer la pression sur ladite ou lesdites têtes.

Accessoirement, la semelle comporte d'une part un élément de correction anti-valgus ou anti-pronation apte à être inséré dans un évidement dit anti-valgus pratiqué sur la face inférieure de la semelle, le long de son bord interne, sous la voûte plantaire, et d'autre part un élément de correction anti-varus ou anti-supination apte à être inséré dans un évidement dit anti-varus pratiqué sur la face inférieure de la semelle, le long de son bord externe, au niveau de la voûte plantaire.

De manière avantageuse, la semelle comporte sur sa face supérieure des moyens guidant le déroulement du pied sur l'axe physiologique de la marche.

Ces moyens consistent en un élément profilé sous-scaphoïdien, sur la face supérieure de la semelle, qui présente sensiblement la forme d'une hémi-coupole et/ou en un élément sous-cuboïdien situé, sur la face supérieure de la semelle, du côté externe de l'élément sous-scaphoïdien et présentant la forme d'une graine de haricot, avec sa convexité tournée vers l'arrière à environ de l'axe longitudinal médian de la semelle et/ou en un moyen axial médio-tarsien, sur la face supérieure de la semelle, de forme ovale et s'élargissant vers l'avant pour se terminer juste devant les têtes métatarsiennes afin de répartir l'appui sous les palettes métatarsiennes.

De préférence, la semelle comporte des moyens pour solliciter dès l'attaque du pas habituellement effectué par le talon, les récepteurs articulaires situés entre l'astragale et le calcanéum.

Lesdits moyens consistent en un canal profilé longitudinal sous-calcanéen, ou console, et d'épaisseur croissante depuis le talon jusqu'à une zone située sensiblement à l'aplomb vertical du col de l'astragale.

Ladite console est positionnée soit sur la face supérieure de la semelle soit sur la face inférieure de la semelle procurant une fonction supplémentaire de raidisseur.

### BREVE DESCRIPTION DES DESSINS

D'autres avantages et caractéristiques ressortiront au mieux de la description qui va suivre de plusieurs variantes d'exécution, données à titres d'exemples non limitatifs, de la semelle orthopédique conforme à l'invention, en référence aux dessins annexés, sur lesquels :
- la figure 1 est une vue de dessous de la semelle orthopédique conforme à l'invention,
- la figure 2 est une vue de côté de la semelle orthopédique suivant l'invention,
- la figure 3 est une vue schématique en coupe longitudinale de la semelle orthopédique avec un pied en appui,
- les figures 4A à 8A sont des vues de dessus des éléments de correction amovibles de la semelle orthopédique suivant l'invention,
- les figures 4B à 8B sont des vues de côté des éléments de correction amovibles de la semelle orthopédique représentés sur les figures 4A à 8A,
- la figure 9 est une vue de dessus d'une variante d'exécution de la semelle orthopédique suivant l'invention,
- la figure 10 est une vue de côté de la variante d'exécution de la semelle orthopédique suivant l'invention représentée sur la figure 9,
- la figure 11 est une vue de dessus d'une seconde variante d'exécution de la semelle orthopédique suivant l'invention,
- la figure 12 est une vue de côté de la seconde variante d'exécution de la semelle orthopédique suivant l'invention représentée sur la figure 11,
- la figure 13 est une vue schématique en coupe longitudinale d'un dispositif de prise d'empreinte pour la détermination de la pointure idoine de la semelle orthopédique suivant l'invention,
- la figure 14 est une vue schématique de dessus du gabarit du dispositif de prise d'empreinte permettant de déterminer la pointure de la semelle en fonction de la position des têtes métatarsiennes par rapport au calcanéum, c'est-à-dire par rapport au talon du patient.

### DESCRIPTION DETAILLEE DE L'INVENTION

Pour des raisons évidentes de simplification de la description, on ne décrira qu'une semelle conforme à l'invention, correspondant au pied gauche par exemple, la semelle droite s'en déduisant par symétrie.

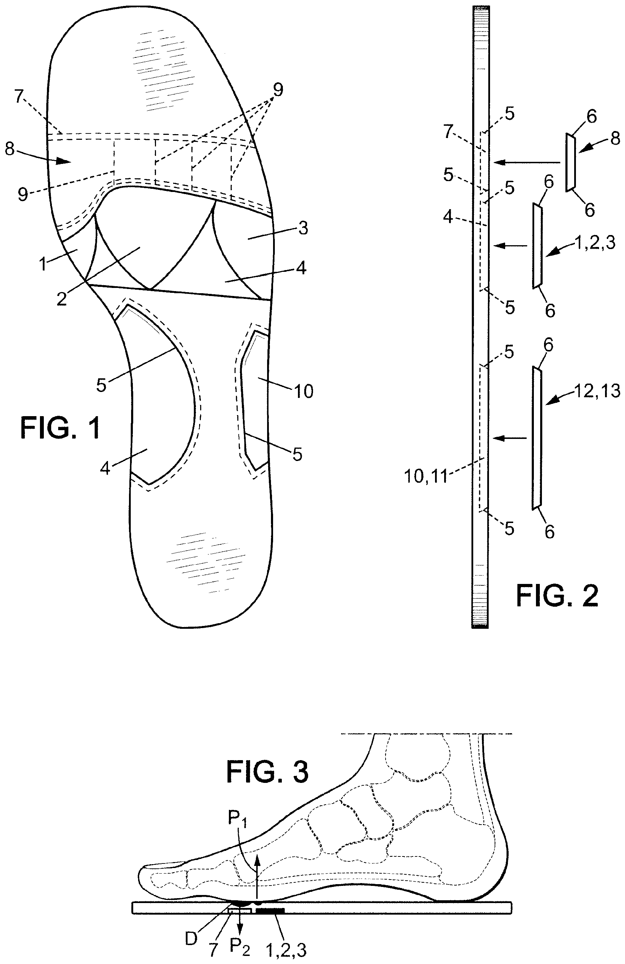

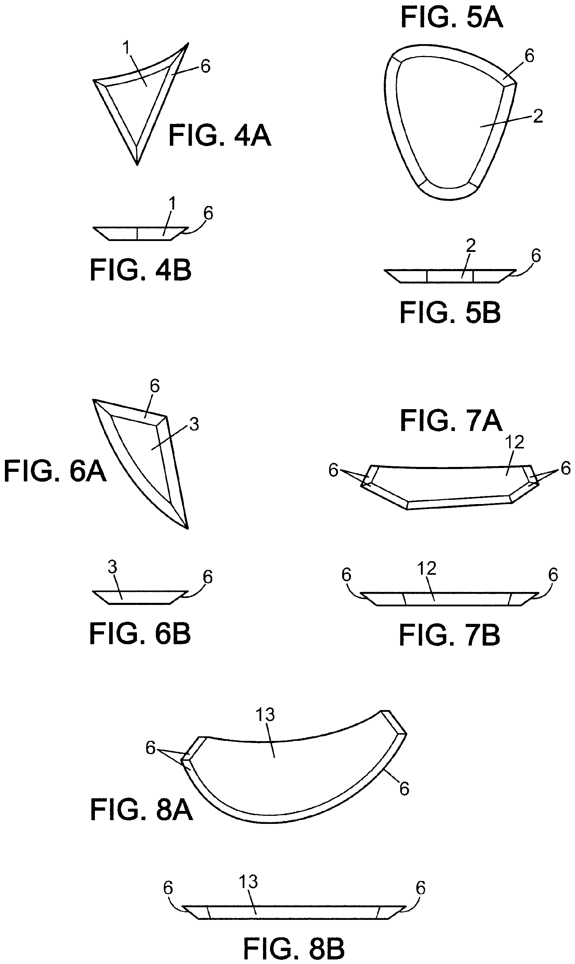

En référence aux figures 1 et 2, la semelle suivant l'invention comporte sur sa face inférieure un élément de correction semi-rigide apte à procurer un appui dit rétro-capital interne 1, sur la partie arrière de la tête du premier métatarse, et/ou un appui rétro-capital dit médian 2, sur la partie arrière de la deuxième et/ou troisième et/ou quatrième tête métatarsienne, et/ou un appui rétro-capital dit externe 3, sur la partie arrière de la cinquième tête métatarsienne. Lesdits éléments de correction rétro-capitaux interne 1, médian 2 et externe 3 sont avantageusement solidarisés à la semelle de manière amovible. La semelle comprend sur sa face inférieure au moins un évidement 4 s'étendant depuis le bord interne jusqu'au bord externe de la semelle, au droit des têtes métatarsiennes, et dans lequel le ou les éléments de correction rétro-capitaux interne 1, médian 2 et externe 3 sont susceptible d'être positionnés. Les parois de l'évidement 4 sont inclinées vers l'intérieur dudit évidement 4 depuis son fond jusqu'au bord dudit évidement, c'est-à-dire jusqu'à la face inférieure de la semelle, afin de former une lèvre 5 au bord de l'évidement 4. Cet évidement 4 est susceptible d'accueillir au moins l'un des éléments de correction rétro-capital interne 1, médian 2 ou externe 3 représentés sur les figures 4A à 6B. Chaque élément de correction rétro-capital 1 à 3 présente une forme sensiblement triangulaire et comprend à sa périphérie un chanfrein 6 de sorte que la lèvre 5 à la périphérie de l'évidement 4 maintienne en place le ou lesdits éléments de correction rétro-capitaux 1 à 3 dans ledit évidement 4.

Il est bien évident que le bord de l'élément de correction rétro-capital interne 1 et de l'élément de correction rétro-capital externe 3 qui est contigu au bord interne et respectivement externe de la semelle lorsque lesdits éléments 1 et 3 sont introduits dans l'évidement 4 ne comprend pas de chanfrein 6.

Par ailleurs, les éléments de correction rétro-capitaux interne 1, médian 2 et externe 3 sont obtenus dans un matériau plus rigide et plus dense que le matériau de la semelle et il est introduit dans l'évidement 4 par déformation élastique de la lèvre 5 de ce dernier.

On notera que la forme particulière de l'évidement 4 et des éléments de correction rétro-capitaux 1 à 3 empêche tout déplacement intempestif, mais également tout retrait, desdits éléments de correction lors de la marche ou de la course.

De plus, on observera que les éléments de correction rétro-capitaux ne sont pas au contact du pied évitant ainsi toute lésion cutanée plantaire telle que des coupures, des ampoules, des crevasses, ou similaires.

De manière avantageuse, en référence aux figures 1 et 2, la semelle comporte sur sa face inférieure un évidement 7 positionné devant les têtes métatarsiennes, au niveau du capiton plantaire, ledit évidement 7 s'étendant depuis le bord interne jusqu'au bord externe de la semelle, et apte à recevoir un élément dit antéro-capital inférieur 8 comportant des lignes de prédécoupes 9 afin de permettre la création d'un creux sous le ou les durillons et/ou affections cutanées. Ces lignes de prédécoupes 9 délimitent des éléments amovibles. Ces éléments amovibles s'étendant au droit des durillons sont retirés afin de créer un creux sur la face inférieure de la semelle. Par ailleurs, de la même manière que précédemment, les parois de l'évidement 7 sont inclinées vers l'intérieur dudit évidement 7 depuis leur fond jusqu'à leur bord respectif afin de former une lèvre 5 en bordure dudit évidement7. Ledit élément antéro-capital inférieur est plat et comprend à sa périphérie, le long de ses bords proximal et distal un chanfrein 6 afin d'assurer son blocage dans l'évidement 7. Dans cet exemple particulier de réalisation, l'élément antéro-capital 8 comporte quatre lignes de prédécoupes 9 s'étendant respectivement du bord proximal au bord distal dudit élément antéro-capital 8.

Ainsi, en référence à la figure 3, les éléments de correction rétro-capitaux 1 à 3 exercent une pression P₁ vers le haut sur la partie arrière de la ou des tête(s) métatarsienne(s) correspondante(s) permettant de corriger les pressions excessives à l'origine de la formation du ou des durillons et le ou les creux 8 formés par retrait des parties amovibles de l'élément antéro-capital 8 entre les lignes de prédécoupes 9 permettent un enfoncement de la semelle dans le creux lorsqu'une pression P₂ (figure 3) est exercée lors de la marche limitant les frottements du durillon D sur la face supérieure de la semelle rendant simultanément la marche beaucoup plus confortable.

L'apparition des durillons étant souvent due à un problème de posture induisant des pressions excessives sur les têtes métatarsiennes, la semelle suivant l'invention comporte avantageusement des moyens de correction de la posture. Ainsi, la semelle, en référence aux figures 1 et 2, comporte, par ailleurs, un second évidement dit anti-varus ou anti-supination 10 de forme globalement rectangulaire, situé le long du bord externe de la semelle et s'étendant depuis le cuboïde jusqu'au premier évidement 4 des éléments de correction rétro-capitaux et un dernier évidement dit anti-valgus ou anti-pronation 11 en forme d'hémi-coupole situé le long du bord interne de la semelle sous la voûte plantaire. De la même manière que précédemment, la paroi des évidements anti-varus 10 et anti-valgus 11 est inclinée vers l'intérieur desdits évidements 10 et 11 depuis leur fond jusqu'à leur bord respectif afin de former une lèvre 5 en bordure desdits évidements 10 et 11. Chacun des évidements anti-varus 10 et anti-valgus 11 est prévu pour accueillir un élément de correction respectivement anti-varus 12 et anti-valgus 13, successivement représentés sur les figures 7A, 7B et 8A, 8B ; l'élément de correction anti-varus 12 a une forme globalement rectangulaire et l'élément de correction anti-valgus 13 à la forme d'une hémi-coupole. Chacun de ces éléments de correction est plat et est obtenu dans un matériau plus rigide et plus dense que le matériau de la semelle; ces éléments comprennent en outre en leur périphérie un chanfrein 6 afin d'assurer leur blocage dans leur évidement respectif.

Selon une variante d'exécution de la semelle, lesdits éléments de correction rétro-capitaux 1,2 et 3, l'élément antéro-capital 8 et les éléments de correction anti-varus 12 et anti-valgus 13 comportent des moyens de fixation aptes à coopérer avec des moyens de fixation complémentaires solidaires de la face inférieure de la semelle.

Ces moyens peuvent consister par exemple dans des ergots faisant saillie de la face supérieure des éléments de correction 1, 2, 3, 8, 12 et 13 et susceptibles de coopérer avec des trous correspondants positionnés au fond des évidements correspondants 4, 8, 10 et 11, les éléments de correction 1, 2, 3, 8, 12 et 13 ne comprenant alors pas de chanfrein 6 et les évidements 4, 8, 10 et 11 ne comprenant pas de lèvre 5.

Alternativement, ces moyens peuvent consister dans des bouclettes et/ou des crochets aptes à coopérer avec des crochets et/ou des bouclettes afin de former des moyens de fixation de type velcro.

Par ailleurs, il est bien évident que les moyens de correction 1, 2, 3, 8, 12 et 13 peuvent être fixés de manière amovible sur la face inférieure de la semelle par tout moyen approprié bien connu de l'homme du métier sans pour autant sortir du cadre de l'invention.

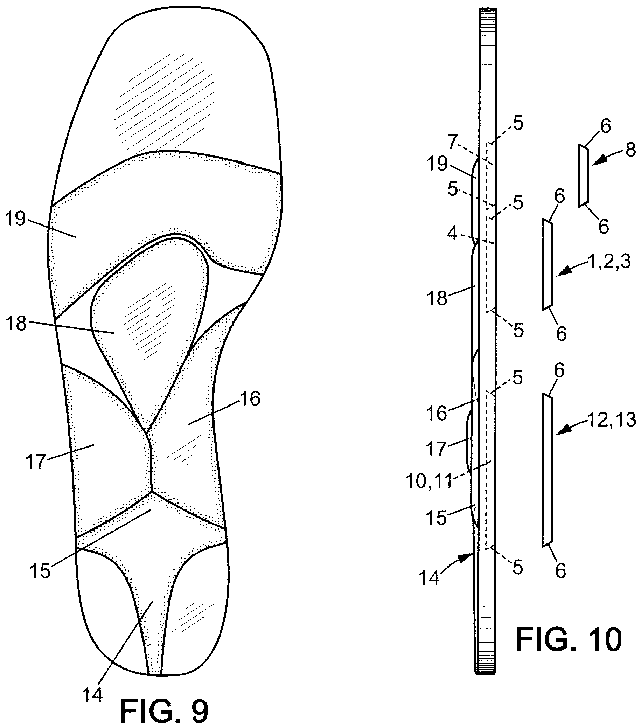

Selon une variante d'exécution particulièrement avantageuse, en référence aux figures 9 et 10, la semelle comporte sur sa face supérieure, des moyens pour solliciter, dès l'attaque du pas habituellement effectué par le talon, les récepteurs articulaires situés entre l'astragale et le calcanéum et des moyens guidant le déroulement du pied sur l'axe physiologique de la marche tels que décrit dans la demande de brevet français FR 2 676 918 déposée par le demandeur. Ces moyens consistent essentiellement dans un canal profilé, appelé console 14, et un jeu d'éléments profilés 16, 17, 18 répartis tout le long de la semelle pour matérialiser un rail autour duquel le pied est guidé.

La console 14 s'étend longitudinalement depuis le talon jusqu'à l'extrémité antérieure du calcanéum, juste à l'aplomb vertical du col de l'astragale. Ladite console 14 présente une épaisseur croissante depuis le talon jusqu'à son extrémité antérieure 15. A titre d'exemple particulier, la hauteur de la console 14 passe progressivement de 1 mm à 2 mm depuis le talon jusqu'à son extrémité antérieure 15.

Le jeu d'éléments profilés 16, 17, 18 est constitué d'arrière en avant, c'est-à-dire depuis le talon vers la pointe du pied, d'un élément profilé sous-scaphoïdien 16, d'un élément profilé sous-cuboïdien 17 et d'un moyen axial médiatarsien 18. L'élément profilé sous-scaphoïdien 16 prolonge la console 14 vers l'intérieur du pied à la manière d'une hémi-coupole. Cet élément profilé sous-scaphoïdien 16 présente dans cet exemple une hauteur d'environ 2 mm et prolonge ainsi l'extrémité antérieure 15 de la console 14.

L'élément profilé sous-cuboïdien 17 se présente en vue de dessus, conformément à la figure 9, sous la forme d'une graine de haricot correspondant globalement à la projection de la forme du cuboïde sur la semelle. Cet élément 17 est situé du côté externe de l'élément sous-scaphoïdien 16, sa convexité tournée vers l'arrière, à environ 45° de l'axe longitudinal médian de la semelle. L'épaisseur dudit élément 17 va, en croissant, du côté vers le centre et d'arrière en avant pour atteindre progressivement une hauteur égale à environ 4 mm.

Le moyen médiatarsien 18 présente une forme ovale, c'est-à-dire grossièrement en forme de goutte d'eau, s'élargissant vers l'avant et se terminant juste devant les têtes métatarsiennes du pied.

Cet élément médiatarsien 18 est bombé; sa hauteur varie longitudinalement depuis une hauteur de 2,5 mm pour atteindre une hauteur maximum de l'ordre de 3,5 mm, à environ deux tiers de sa longueur. On observera que, lors du déroulement du pas, la console 14 sollicite le calcanéum du pied, qu'il s'agisse d'un pied plat ou d'un pied creux, pour préparer dans de bonnes conditions la suite du pas; puis les éléments profilés sous-scaphoïdien 16 et/ou sous-cuboïdien 17 qui agissent en stabilisateurs latéraux du pied, incitent le pied à rester dans le rail physiologique de la marche et l'élément médiatarsien 18 prépare la phase digitigrade terminale du pas en répartissant l'appui du pied sous les palettes métatarsiennes de manière à ce que cet appui reste canalisé sur l'axe du deuxième métatarsien par lequel passe l'axe physiologique de la marche.

De manière avantageuse, la semelle comporte sur sa face supérieure un élément antéro-capital supérieur dit capiton plantaire supérieur 19 s'étendant depuis le bord interne jusqu'au bord externe de la semelle devant l'élément médiatarsien, présentant une forme générale de haricot, et obtenu dans une matière souple afin de procurer un amorti lors de la marche. Ce capiton plantaire supérieur 19 présente une hauteur comprise entre 2 et 4 mm et est obtenue dans toute matière souple bien connue de l'homme du métier.

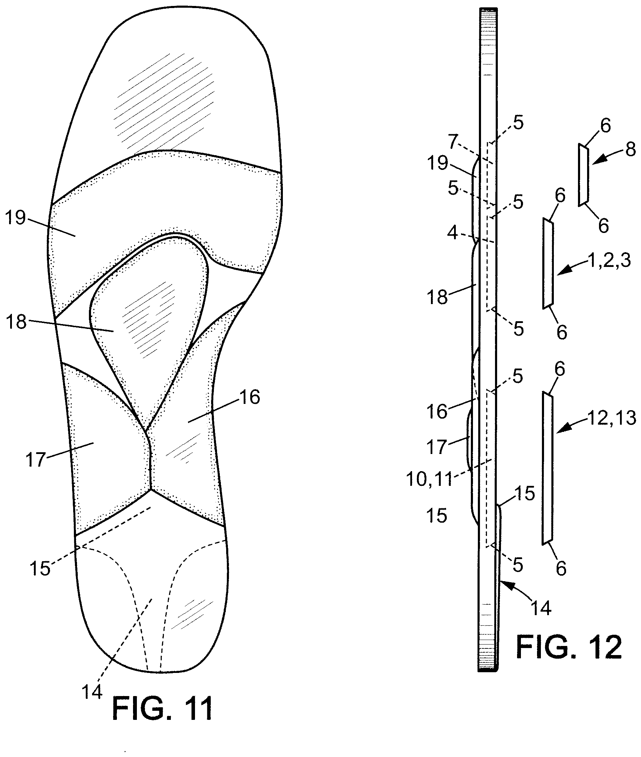

Selon une dernière variante d'exécution particulièrement avantageuse, en référence aux figures 11 et 12, la semelle comporte des moyens pour solliciter, dès l'attaque du pas habituellement effectué par le talon, les récepteurs articulaires situés entre l'astragale et le calcanéum et des moyens guidant le déroulement du pied sur l'axe physiologique de la marche. Ces moyens consistent essentiellement dans un canal profilé, appelé console 14, et un jeu d'éléments profilés sous-scaphoïdien 16, sous-cuboïdien 17 et médiotarsien 18 répartis tout le long de la semelle pour matérialiser un rail autour duquel le pied est guidé. De plus, la semelle comporte de la même manière que précédemment un capiton plantaire supérieur 19. Cette variante d'exécution diffère de la précédente par le fait que la console 14 est positionnée sur la face inférieure. De cette manière, outre sa fonction de sollicitation des récepteurs articulaires lors de l'attaque du pas, la console 14 présente une fonction de raidisseur.

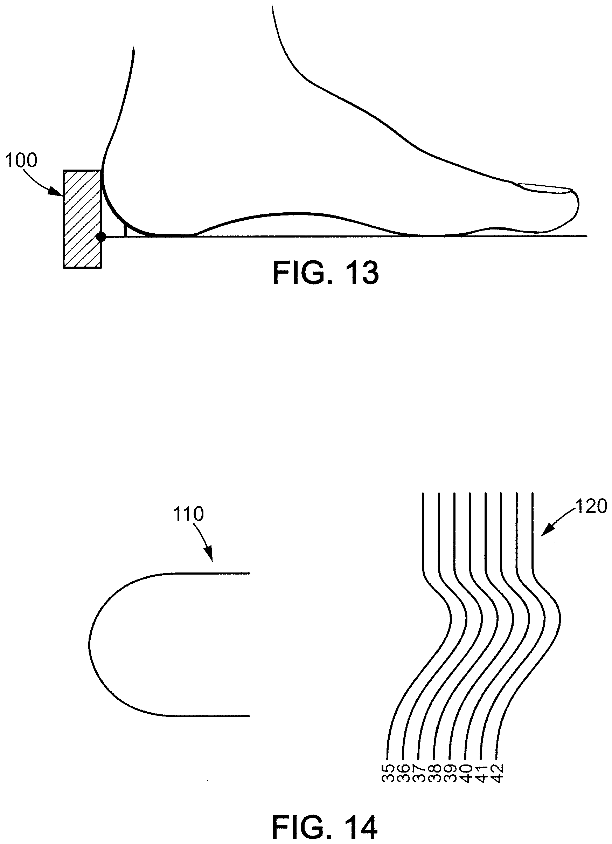

Afin de permettre la fourniture rapide de semelle aux patients, un dispositif de prise d'empreinte classique 100, en référence aux figures 13 et 14, permet une prise d'empreinte par transfert d'encre ou par déformation d'une mousse par exemple bien connu de l'homme du métier, et d'un gabarit 110 transparent ou translucide comportant des lignes prédéfinies 120 correspondant à la position des têtes métatarsiennes pour différentes pointures. Ainsi, il suffit de prendre l'empreinte du patient puis de faire correspondre la partie postérieure du gabarit sur la partie postérieure talonnière de l'empreinte ainsi relevée et de lire la pointure correspondante. Comme la partie postérieure de l'empreinte est en avant de 6 mm par rapport à la partie postérieure du talon, le gabarit a été conçu en prenant en compte cette différence. Il suffit alors d'ajouter le ou les éléments de correction rétro-capitaux 1 et/ou 2 et/3 dans l'évidement 4 de la semelle correspondant à la pointure relevée et de découper l'élément antéro-capital inférieur 8 de la semelle au niveau des lignes de découpe 9, si nécessaire, afin de réaliser une semelle correctrice pour supprimer le ou les durillons du patient. Le ou lesdits durillons disparaissent en un mois environ et la correction apportée permet d'éviter toute apparition de nouveaux durillons.

Enfin, il est bien évident que les exemples que l'on vient de donner ne sont que des illustrations particulières en aucun cas limitatives quant aux domaines d'application de l'invention.

## Revendications

1. Semelle orthopédique intérieure apte à être insérée dans une chaussure ou similaire pour soulager et/ou traiter des affections cutanées et/ou des durillons et/ou des douleurs métatarsiennes, comprenant sur sa face inférieure au moins un évidement (4) s'étendant depuis le bord interne jusqu'au bord externe de la semelle, au droit des têtes métatarsiennes, et dans lequel au moins un élément de correction (1, 2, 3) est positionné de manière amovible,
**caractérisée en ce que** ledit élément de correction est formé d'un matériau plus rigide et plus dense que le matériau de la semelle et est apte à
procurer un appui dit rétro-capital interne (1), sur la partie arrière de la tête du premier métatarse, et/ou un appui rétro-capital dit médian (2), sur la partie arrière de la deuxième et/ou troisième et/ou quatrième tête métatarsienne, et/ou un appui rétro-capital dit externe (3), sur la partie arrière de la cinquième tête métatarsienne..

2. Semelle suivant la revendication 1, **caractérisée en ce qu'**elle comporte sur sa face inférieure un évidement (7) positionné devant les têtes métatarsiennes, au niveau du capiton plantaire, ledit évidement (7) s'étendant depuis le bord interne jusqu'au bord externe de la semelle, et apte à recevoir un élément dit antéro-capital inférieur (8) comportant des lignes de prédécoupes (9) afin de permettre la création d'au moins un creux sous le ou les durillons et/ou affections cutanées.

3. Semelle suivant l'une quelconque des revendications 1 ou 2, **caractérisée en ce qu'**elle comporte un élément de correction anti-valgus ou anti-pronation (13) apte à être inséré dans un évidement dit anti-valgus (1 1) pratiqué sur la face inférieure de la semelle, le long de son bord interne, sous la voûte plantaire.

4. Semelle suivant l'une quelconque des revendications 1 à 3, **caractérisée en ce qu'**elle comporte un élément de correction anti-varus ou anti-supination (12) apte à être inséré dans un évidement dit anti-varus (10) pratiqué sur la face inférieure de la semelle, le long de son bord externe, au niveau de la voûte plantaire.

5. Semelle suivant l'une quelconque des revendications 1 à 4, **caractérisée en ce qu'**elle comporte sur sa face supérieure des moyens (14,16,17,18) guidant le déroulement du pied sur l'axe physiologique de la marche.

## Patentansprüche

1. Orthopädische Einlegesohle zum Einlegen in einen Schuh oder ähnliches, um Hauterkankungen und/oder Schwielen und/oder Schmerzen im Mittelfuß zu lindern und/oder zu behandeln, welche an ihrer Unterseite mindestens eine Aussparung (4) aufweist, die sich in Höhe des Mittelfußkopfes vom Innenrand bis zum Außenrand der Sohle erstreckt und in der mindestens ein Korrekturelement (1, 2, 3) abnehmbar positioniert ist, **dadurch gekennzeichnet, dass** das Korrekturelement aus einem Material gebildet wird, das steifer und dichter als das Material der Sohle ist und eine sogenannte innere retrokapitale Stützung (1) im hinteren Bereich des Kopfes des ersten Mittelfußes bilden kann, und/oder eine sogenannte mediane retrokapitale Stützung (2) im hinteren Bereich des zweiten und/oder dritten und/oder vierten Mittelfußkopfes, und/oder eine sogenannte äußere retrokapitale Stützung (3) im hinteren Bereich des fünften Mittelfußkopfes.

2. Sohle nach Anspruch 1, **dadurch gekennzeichnet, dass** sie an ihrer Unterseite eine Aussparung (7) aufweist, die vor dem Mittelfußkopf am Fußballen positioiert ist, wobei sich besagte Aussparung (7) vom Innenrand bis zum Außenrand der Sohle erstreckt, und ein sogenanntes anterokapitales unteres Element (8) aufnehmen kann, das vorgestanzte Linien (9) aufweist, um die Bildung von mindestens einem Hohlraum unter der oder den Schwielen und/oder Hauterkrankungen zu ermöglichen.

3. Sohle nach einem der Ansprüche 1 oder 2, **dadurch gekennzeichnet, dass** sie ein antivalgisches oder antipronatorisches Korrekturelement (13) aufweist, das in eine sogenannte antivalgische Aussparung (11), die an der Unterseite der Sohle entlang des Innenrands unter der Fußssohle eingearbeitet ist, eingesetzt werden kann.

4. Sohle nach einem der Ansprüche 1 bis 3, **dadurch gekennzeichnet, dass** sie ein antivarisches oder antisupinatorisches Korrekturelement (12) enthält, das in eine sogenannte antivarische Aussparung (10) an der Unterseite der Sohle entlang dem Außenrand in Höhe der Fußssohle eingesetzt werden kann.

5. Sohle nach einem der Ansprüche 1 bis 4, dadurch gekenzeichnet, dass sie an der Oberseite Mittel (14, 16, 17, 18) aufweist, die den Abrollvorgang des Fußes auf der physiologischen Gangachse führen.

## Claims

1. An orthopedic insole capable of being inserted into a shoe or the like for relieving and/or treating skin disorders and/or calluses and/or metatarsal pain, comprising, on the lower face thereof, at least one recess (4) extending from the inner edge to the outer edge of the sole, at the metatarsal heads, and wherein at least one corrective element (1, 2, 3) is removably placed, **characterized in that** said corrective element is made of a more rigid and denser material than the material of the sole and is capable of providing a so-called internal retro-capital support (1), on the back portion of the head of the first metatarsus, and/or a so-called median retro-capital support (2), on the back portion of the second and/or third and/or fourth metatarsal head(s), and/or a so-called external retro-capital support (3), on the back portion of the fifth metatarsal head.

2. A sole according to claim 1, **characterized in that** it comprises, on the lower face thereof, a recess (7) positioned in front of the metatarsal heads, at the footpad, with said recess (7) extending from the inner edge to the outer edge of the sole, and being capable of receiving a so-called lower antero-capital element (8) provided with precut lines (9) so as to enable the creation of at least one recess under the callus(es) and/or skin disorders.

3. A sole according to any one of claims 1 or 2, **characterized in that** it comprises an anti-valgus or anti-pronation corrective element (13) capable of being inserted into a so-called anti-valgus recess (11) provided on the lower face of the sole, along the inner edge thereof, under the plantar arch.

4. A sole according to any one of claims 1 to 3, **characterized in that** it comprises an anti-varus or anti-supination corrective element (12) capable of being inserted into a so-called anti-varus recess (10) provided on the lower face of the sole, along the outer edge thereof, at the plantar arch.

5. A sole according to any one of claims 1 to 4, **characterized in that** it comprises, on the upper surface thereof, means (14, 16, 17, 18) for guiding the movement of the foot along the physiological axis of the walk.
